# EUROPEAN PATENT SPECIFICATION

(11) **EP 1 169 025 B1**
(45) Date of publication and mention of the grant of the patent: **24.08.2005**
(21) Application number: 00918530.7
(22) Date of filing: 31.03.2000
(51) Int. Cl.: A61K 9/70, A61K 47/34

(54) **TRANSDERMAL DRUG DELIVERY DEVICES COMPRISING A POLYURETHANE DRUG RESERVOIR**
TRANSDERMALE VORRICHTUNG ZUR ARZNEISTOFFVERABREICHUNG MIT EINEM POLYURETHANWIRKSTOFFRESERVOIR
DISPOSITIFS D'ADMINISTRATION TRANSDERMALE DE MEDICAMENTS COMPRENANT UN RESERVOIR DE MEDICAMENT EN POLYURETHANE

(30) Priority: 01.04.1999 US 127412 P
(43) Date of publication of application: 09.01.2002
(73) Proprietor: ALZA CORPORATION, Mountain View, CA 94039-7210 (US)
(72) Inventor: VENKATRAMAN, Subramanian, S., Palo Alto, CA 94303 (US); STEIN, Thomas, M., San Jose, CA 95124 (US); SNIDER, James, Newark, CA 94560 (US); HAMLIN, Richard, D., Newark, CA 94560 (US)
(74) Representative: Suèr, Steven Johannes
(86) International application number: PCT/US2000/008670
(87) International publication number: WO 2000/059483

(56) References cited:
- WO-A-91/05809
- WO-A-97/09970
- US-A- 5 338 548
- US-A- 5 505 958

## Description

### Field of the Invention

The present invention relates to the field of transdermal drug delivery. More specifically, the present invention relates to methods of making drug reservoir materials for use in transdermal drug delivery devices. The drug reservoirs comprise a polyurethane polymer that can be processed at temperatures below those that cause degradation of temperature sensitive drugs and/or excipients. The release characteristics of the polyurethane material may be tailored in order to accommodate a range of suitable drugs to be delivered from the transdermal drug delivery device and/or provide a range of delivery rates for a particular drug.

### Background of the Invention

The transdermal route of parenteral drug delivery provides many advantages over other administrative routes. Transdermal drug delivery devices for delivering a wide variety of drugs or other beneficial agents are described in U.S. Patent Nos. 3,598,122; 3,598,123; 3,731,683; 3,797,494; 4,031,894; 4,201,211; 4,286,592; 4,314,557; 4,379,454; 4,435,180; 4,559,222; 4,568,343; 4.588,580; 4,645,502; 4,698,062; 4,704,282; 4,725,272; 4,781,924; 4,788,062; 4,816,258; 4,849,226; 4,904,475; 4,908,027; 4,917,895; 4,938,759; 4,943,435; 5,004,610; 5,071,656; 5,141,750; 5,342,623; 5,411,740; and 5,635,203.

Transdermal drug delivery devices typically fall into one of three categories:
1) Form-Fill-Seal systems wherein the drug is contained within a gel reservoir layer which fills a cavity defined between a rate controlling membrane and a backing layer;
2) Multilaminate systems defined as a transdermal system in which the drug and excipient are formulated into a layer distinct from the adhesive layer. As the name implies the system may consist of two or more layers in addition to the backing layer; and
3) Matrix/monolith (also called "drug-in-adhesive") system defined as a transdermal system in which the drug and excipients are formulated directly into an adhesive; the adhesive and backing make up the system.

Form-Fill-Seal systems and their manufacture are described in U.S. Patent No. 4,379,454, for example. These devices are generally more difficult to manufacture and tend to be bulkier than multilaminates and matrix devices. Thus, systems of this type are less attractive for both manufacturing and cosmetic reasons.

Multilaminate systems are typically manufactured by a solvent casting process as described in U.S. Patent No. 4,286,592, for example, wherein the drug, permeation enhancer, and/or polymeric carrier are mixed with an organic solvent and cast onto a substrate such as a backing layer, rate control membrane, or release liner. The film is then heated to drive off the organic solvent. At least two films are cast (i.e. the drug reservoir and adhesive films) and subsequently laminated together to form a final multilaminate device.

Monoliths are manufactured in a manner similar to the multilaminate process (i.e. solvent casting the drug, excipient, and adhesive polymer components), but consist of a single film. In addition to the solvent casting process described above, hot-melt processing has been disclosed as a process for manufacturing transdermal drug delivery devices as disclosed in, for example, U.S. Patent Nos. 5,273,757, 5,536,759, 5,641,506, 5,662,923, 5,662,926, 5,670,164, and 5,688,523, hereby incorporated in their entirety by reference. However, both solvent casting and hot-melt processing methods have their drawbacks.

Solvent casting of the polymer layers for transdermal drug delivery systems requires dissolving and/or suspending the drug and/or excipients in a solvent, coating the resulting solution onto a web, then oven drying the coated web to evaporate the solvent from the cast film. Residual solvent must be at a very low concentration since the film is intended for skin contact applications. However, as seen in the above cited patents, the use of solvents in the manufacture of the various layers of transdermal systems is disadvantageous for several reasons.

First, solvent casting requires additional expenses for the solvents, drying and extraction equipment, and even further costs associated with the recovery, separation, or incineration of the solvents. Secondly, the removal of solvent requires the application of elevated temperatures to the polymer film, which can strip the lower molecular weight components, including the drug, from the film and also cause degradation of drug and/or excipient. The temperature may be lowered, in which case the time required to evaporate the solvent is substantially increased. Additionally, the flammability of the solvents and the risk of harm organic solvents pose to human organisms raise additional concerns.

Hot-melt processing, while avoiding the need to remove any solvent, still subjects the polymer, drug, and possible excipients to elevated temperatures and further suffers from higher mixing torques. For example, U.S. Patent Nos. 5,536,759 and 5,662,923 cited above disclose processing temperatures for hot-melt adhesives ranging from 60 °C to 180 °C. U.S. Patent No. 5,662,926 listed above discloses transdermal patches incorporating a drug containing polymer film processed at temperatures of 170 - 200 °C. Such elevated temperatures may cause degradation of drug and/or excipients, particularly if they are heat sensitive. Further, such processes also take time, typically in excess of 0.5 hours, to complete mixing.

Various materials, including polyurethanes, have been proposed for use as drug reservoirs in transdermal drug delivery devices. For example, ethylene vinylacetate (EVA) copolymers are disclosed in U.S. Patent No. 4,144,317 as a suitable drug reservoir material for transdermal drug delivery applications. However, EVA has a limited solubility range for drugs, as the composition can vary from only about 5% vinyl acetate (VA) to about 40% VA before permeability is compromised for any drug. Loading the reservoir beyond saturation with drug and/or permeation enhancer, which is typically required for EVA 40, leads to phase separation. Most permeation enhancers are surfactant in nature and tend to migrate to interfaces. Phase separation and this migration tendency in turn may lead to delamination of the drug reservoir from the backing and/or adhesive layer(s).

In general, polyurethanes are copolymers of a "hard" segment having a high glass transition temperature (Tg) and a "soft" segment (low Tg). The hard segment is typically a diisocyanate such as methylene bis(cyclohexyl) diisocyanate (HMDI) that is chain-extended with a diol, such as 1,4 butane diol. For polyether polyurethanes, the soft segments are typically a polyether alcohol such as poly tetramethylene glycol (PTMEG), poly propylene glycol (PPG), and/or polyethylene glycol (PEG). If the soft segment is composed of PEG chains, the polyurethane will be very hydrophilic; on the other hand, soft segments made of aromatic polyether (PTMEG) will yield hydrophobic polyurethanes.

One problem associated with polyurethanes of the prior art is their high processing temperature, typically 170 - 250 °C. These high temperatures can cause degradation of temperature sensitive drugs and/or excipients. This precludes melt-mixing of most drugs into the polyurethane polymer to obtain a drug reservoir for a transdermal drug delivery device.

Polyurethanes for use in transdermal drug delivery devices are known in the art. For example, US Patent No. 4,523,005 discloses polyurethane polymers formed from a diisocyanate, a high molecular weight polyether polyol, and a low molecular weight glycol chain extender (1,4 butane diol). The polyol should have a molecular weight between 500 - 5000, PTMEG is preferred. As seen in the examples, the polyurethane polymer pellets may be extruded at temperatures of about 170 °C.

U.S. Patent No. 4,638,043 discloses a drug releasing system comprised of a drug dispensing polyurethane member as a matrix for a therapeutically effective amount of a drug dispersed therein. The polyurethane is a polyurethane acrylic copolymer which is the reaction product of a diisocyanate, a glycol with a molecular weight between the range of about 500 - 5000, and an acrylyl chain terminator having a molecular weight between the range of 40 - 200 cured by actinic radiation. These polyurethanes are further disclosed in U.S. Patent No. 4,483,759.

Re 32,991 discloses a polyurethane being the reaction product of a diisocyanate, a macroglycol, and a chain terminator (HEMA). The macroglycol comprises a glycol having a molecular weight in excess of 500 Daltons, and is preferably PPG or PEG.

US Patent No. 4,638,043 discloses polyurethanes comprising polycarbonate glycols having a molecular weight between 500 - 2000 as the preferred macroglycol.

US Patent No. 5,569,683 discloses polyurethane gel compositions that may include glycols such as propylene glycol, PPG, and PEG.

US Patent No. 4,746,509 discloses transdermal drug delivery devices comprising homogeneous membranes of variable hydrophilicity which enable the kinetics of the release of drug to be controlled. Heat-reversible, non-cross-linked, film-forming polymers are used which are insoluble in water but may be water-miscible and in which it is possible to vary the hydrophilic character. More particularly, polyurethane/polyoxyethylene glycol/polyoxypropylene glycol copolymers are disclosed as suitable for practice of the invention.

US Patent No. 5,118,779 discloses a hydrophilic polyurethane polymer which is polymerizable in the substantial absence of heat wherein the urethane prepolymer comprises a diisocyanate, a bifunctional component having at least one active hydrogen on each terminal group at least a portion of which is polyethyleneoxide, and a chain extender. The polyethyleneoxide containing material provides hydrophilicity to the polyurethane polymer and has a molecular weight from about 500 - 3000 Daltons. The balance of this component may be another macroglycol such as PPG or PTMEG. The amount of polyethyleneoxide containing material may range from 5 - 85% by weight of the final elastomer, depending on the desired hydrophilicity.

Despite the advances in the art, there remains a need in the transdermal drug delivery art to provide a polymer film layer into which the drug is dissolved and/or suspended without the use of solvents. Additionally, the need remains for "tunable" membranes and/or drug reservoirs in transdermal drug delivery and manufacturing processes thereof which overcome the above problems associated with the prior art.

### Description of Terms

As used herein, the term "drug" is to be construed in its broadest sense to mean any material which is intended to produce some biological, beneficial, therapeutic, or other intended effect, such as permeation enhancement, for example, on the organism to which it is applied

As used herein, the term "individual" intends a living mammal and includes, without limitation, humans and other primates, livestock and sports animals such as cattle, pigs and horses, and pets such as cats and dogs.

As used herein, the term "monoglyceride" refers to a monoglyceride or mixture of monoglycerides of C₈₋₂₀ fatty acids and includes, without limitation, glycerol monolaurate (GML), glycerol monooleate (GMO), glycerol monocaprate (GMC), glycerol monocaprylate (GMCL), and glycerol monolinoleate (GMLO).

As used herein, the term "permeation enhancement" intends an increase in the permeability of skin in the presence of a permeation enhancer as compared to permeability of skin in the absence of a permeation enhancer.

As used herein, the term "permeation enhancer" intends an agent or a mixture of agents which acts to increase the permeability of the skin to a drug

As used herein, the term "permeation-enhancing amount" intends an amount of a permeation enhancer which provides permeation enhancement throughout a substantial portion of the administration period.

As used herein, the phrase "predetermined area of skin" intends a defined area of intact unbroken skin or mucosal tissue. That area will usually be in the range of about 5 cm² to about 100 cm².

As used herein, the phrase "sustained time period" or "administration period" intends at least about 8 hours and will typically intend a period in the range of about one to about seven days, preferably about 1 - 3 days.

As used herein, the term "transdermal" intends both percutaneous and transmucosal administration, i.e., passage of drug through a body surface or membrane such as intact unbroken skin or mucosal tissue into the systemic circulation.

### Summary of the invention

According to one aspect of this invention, there is provided a method of making a reservoir matrix material for a transdermal drug delivery device, which method comprises the steps of:
(a) providing a drug;
(b) providing a polyurethane polymer; and
(c) melt-blending the drug into the polyurethane polymer at a temperature of less than 150°C. The polyurethanes can be made to be essentially amorphous and the compositions can be varied over a broad compositional range. By manipulating the ratio of the "hard" and "soft" segments of the polyurethane, a range of permeabilities for a given drug may be obtained. Additionally, by changing the nature of the soft segments, materials of different hydrophobic/hydrophilic nature or drug solubilities can be obtained. The polyurethanes can be melt-blended with the drug at temperatures lower than 150°C, preferably lower than 100°C, and most preferably within 40-90°C without the use of organic solvents.

The polyurethane polymer may be a polyether polyurethane. The polyurethane polymer may comprise the reaction product of at least one aliphatic diisocyanate, at least one polyether polyol, and at least one glycol. The diisocyanate may comprise methylene bis(cyclohexyl)diisocyanate, and the polyether polyol may comprise polytetramethylene ether glycol, polypropylene glygol, or polyethylene glycol. The glycol may be 1,4-butane diol. The polyether polyol may be a mixture of at least two polymers selected from the group consisting of polytetramethylene ether glycol, polypropylene glycol, polyethylene glycol and propylene glycol.

The matrix material may have a thickness of 25.4 to 304.8 µm (1 to 12 mils). Preferably, the thickness of the matrix material is 50.8 to 152.4 µm (2 to 6 mils).

The matrix material may have a room-temperature modulus between 0.1 and 100 MPa.

The matrix material may further contain at least one permeation enhancer in an amount of up to 20 wt%. Preferably, the matrix material contains 2 - 15 wt% of a permeation enhancer.

The permeation enhancer may be selected from the group consisting of monoglycerides and lauryl pyroglutamate. Preferably, the permeation enhancer is glycerol monolaurate, or lauryl pyroglutamate.

The matrix material may comprise 0.1 - 40 wt% of the drug.

The drug may be selected from the group consisting of fentanyl, oxbutynin, and fluoxetine. Preferably, the matrix material contains 1 - 10 wt% fentanyl base. More preferably, the matrix material contains 4 - 7 wt% fentanyl base, 4 - 13 wt% of a permeation enhancer, and 75 - 92 wt% of a polyether polyurethane.

Also described are materials for use in transdermal drug delivery systems.

Also described are polyurethanes for transdermal drug delivery applications which offer a range of permeabilities for a drug to be transdermally administered.

Also described are more easily processed polyurethanes for transdermal drug delivery applications.

Also described are drug reservoirs and membranes for transdermal drug delivery devices which can be processed at process temperatures of less than about 150°C, preferably less than about 100°C, most preferably within about 40-90°C without the use of organic solvents.

Also described are matrix materials for transdermal drug delivery applications which are substantially free of residual solvent.

These and other aspects of this invention will be made clear from the description that follows, which is understood to include equivalents thereof and is not to be limited by the specific disclosure and examples.

### Brief Description of the Drawings

Figure 1 is a cross-section through a schematic perspective view of a transdermal therapeutic system comprising a reservoir matrix material obtainable by a method according to this invention.
Figure 2 is a cross-section view through another transdermal therapeutic system comprising a reservoir matrix material obtainable by a method according to this invention prior to application to the skin.
Figure 3 is a cross-section view through another transdermal therapeutic system comprising a reservoir matrix material obtainable by a method according to this invention prior to application to the skin.
Figure 4 depicts the release rate of fentanyl from devices comprising polyurethane reservoirs obtainable by a method according to this invention.

### Detailed Description

The present invention utilises polyurethanes particularly suited for transdermal drug delivery devices and applications. The polyurethanes used in the invention provide a greater degree of versatility as variations in processibility, stability, and drug permeability are all enhanced. The polyurethanes used in the present invention may be processed into films for incorporation into transdermal drug delivery devices at temperatures less than about 150 °C without the use of organic solvents so that drug(s) and/or excipients can be directly incorporated into the polyurethane polymer by melt-mixing. The polyurethanes can be used as drug reservoirs as well as rate controlling membranes for transdermal drug delivery devices. Permeation of both hydrophilic and hydrophobic drugs can be controlled using the polyurethanes described.

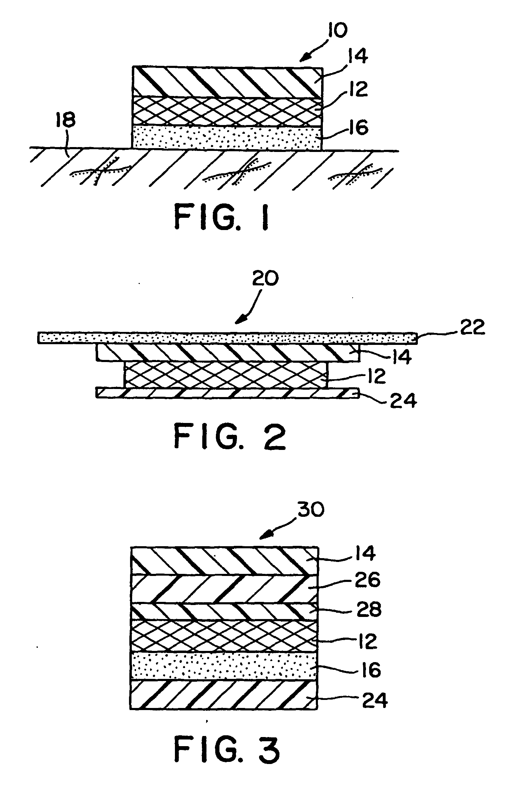

Referring to Figure 1, a transdermal therapeutic system comprises transdermal delivery device 10 comprising a polyurethane drug reservoir 12 containing at least one drug and/or a permeation enhancer dispersed and/or dissolved therein. Reservoir 12 is sandwiched between a backing 14 and an in-line contact adhesive layer 16. The device 10 adheres to the surface of the skin 18 by means of the adhesive layer 16. The adhesive layer 16 may optionally contain the permeation enhancer and/or drug. A removable release liner (not shown in FIG. 1) is normally provided along the exposed surface of adhesive layer 16 and is removed prior to application of device 10 to the skin 18. Optionally, a rate-controlling membrane (not shown) may be present between the reservoir 12 and the adhesive layer 16. Additionally, a non-rate controlling tie layer membrane as disclosed in US Patent No. 5,635,203, incorporated herein in its entirety by reference, may be present between the reservoir 12 and adhesive 16 in any of the embodiments depicted in Figures 1- 3.

Although the transdermal therapeutic system shown in Figure 1 utilizes an in-line adhesive, other means for maintaining the system on the skin can be employed. Such means include a peripheral ring of adhesive outside the path of the drug from the system to the skin or the use of other fastening means such as buckles, belts, and elastic arm bands.

Alternatively, as shown in FIG. 2, transdermal therapeutic device 20 may be attached to the skin or mucosa of a patient by means of an adhesive overlay 22. Device 20 is comprised of polyurethane drug reservoir 12 containing at least one drug and/or a permeation enhancer dispersed and/or dissolved therein. A backing layer 14 is provided adjacent to one surface of reservoir 12. Adhesive overlay 22 maintains the device on the skin and may be fabricated together with, or provided separately from, the remaining elements of the device. With certain formulations, the adhesive overlay 22 may be preferable to the in-line contact adhesive 16 as shown in FIG. 1. Backing layer 14 is preferably slightly larger than reservoir 12, and in this manner prevents the materials in reservoir 12 from adversely interacting with the adhesive in overlay 22. Optionally, a rate-controlling membrane (not shown in FIG. 2) may be provided on the skin-proximal side of reservoir 12. A removable release liner 24 is also provided with device 20 and is removed just prior to application of device 20 to the skin.

In FIG. 3, transdermal delivery device 30 comprises a polyurethane drug and permeation enhancer reservoir ("drug reservoir") 12 substantially as described with respect to FIG. 1. Permeation enhancer reservoir ("enhancer reservoir") 26 comprises the permeation enhancer dispersed throughout and contains drug at or below saturation, when in equilibrium with the drug reservoir 12. Enhancer reservoir 26 is preferably made from substantially the same matrix as is used to form drug reservoir 12. A rate-controlling membrane 28 for controlling the release rate of the permeation enhancer from enhancer reservoir 26 to drug reservoir 12 is placed between the two reservoirs. A rate-controlling membrane (not shown in FIG. 3) for controlling the release rate of the enhancer and/or drug from drug reservoir 12 to the skin may also optionally be utilized and would be present between adhesive layer 16 and reservoir 12.

Superimposed over the permeation enhancer reservoir 26 of device 30 is a backing 14. On the skin-proximal side of reservoir 12 are an adhesive layer 16 and a removable liner 24 which would be removed prior to application of the device 30 to the skin.

It is preferable for the devices depicted in Figures 1 - 3 to control the rate at which the drug is released from the device to the skin or mucosa of a host. The in-line contact adhesive 16 is capable of controlling the rate at which the drug is released to the skin or mucosa surface. Alternatively, a rate controlling membrane is utilized for this purpose as set forth above. The rate-controlling membrane may be fabricated from permeable, semipermeable or microporous materials which are known in the art to control the rate of agents into and out of delivery devices and having a permeability to the permeation enhancer lower than that of drug reservoir 12. Suitable materials include, but are not limited to, polyethylene, polyvinyl acetate, ethylene n-butyl acetate and ethylene vinyl acetate copolymers. The polyurethane drug reservoir layer can control the rate at which drug is released from the system as discussed in greater detail below. Polyurethanes can also be used as rate controlling membranes distinct from the drug reservoir.

Permeation enhancers suitable for practice of this invention may be any permeation enhancer known in the art to increase permeability of drugs through skin and includes, but is not limited to, those disclosed in the above cited patents. Preferably, the permeation enhancer comprises a permeation enhancing amount of a permeation enhancer including, but not limited to monoglycerides, C₁₀- C₂₀ fatty acid esters including ethyl palmitate and isopropyl myristate; acyl lactylates such as caproyl lactylic acid and lauroyl lactylic acid; dimethyl lauramide; dodecyl (lauryl) acetate; lactate esters such as lauryl lactate, and myristyl lactate, monoalkyl ethers of polyethyleneglycol and their alkyl or aryl carboxylic acid esters and carboxymethyl ethers such as polyethylene glycol-4 lauryl ether (Laureth-4) and polyethylene glycol-2 lauryl ether (Laureth-2); Myreth-3, myristyl sarcosine, and methyl laurate.

The backing material is selected from materials known in the art and is preferably an occlusive film. Preferred materials include multilaminate films including, but not limited to medium density polyethylene (MDPE) / polyester *l* aluminum / ethylene vinylacetate (EVA) laminates, polyolefin/polyurethane films such as low density polyethylene/polyurethane laminates, or medium density polyethylene / polyurethane laminates.

Adhesives suitable for use are known in the art as disclosed , for example, in the above cited patents and are preferably pressure sensitive adhesives. Such pressure sensitive adhesives include, but are not limited to, polysiloxanes, polyacrylates, polyurethanes, acrylic adhesives including cross linked or uncross linked acrylic copolymers, vinyl acetate adhesives, ethylene vinylacetate copolymers, and natural or synthetic rubbers including polybutadienes, polyisoprenes, and polyisobutylene adhesives, and mixtures and graft copolymers thereof

It is believed that this invention has utility in connection with the delivery of drugs within the broad class normally delivered through body surfaces and membranes, including skin. In general, this includes therapeutic agents in all of the major areas, including, but not limited to, ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, antipyretics and anti-inflammatory agents, androgens, local and general anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretic, antiemetic and prokinetic agents, antiepileptic agents, antiestrogens, antifungal agents, antihypertensive agents, antimicrobial agents, antimigraine agents, antimuscarinic agents, antineoplastic agents, antiparasitic agents, antiparkinson's agents, antiplatelet agents, antiprogestins, antithyroid agents, antitussives, antiviral agents, atypical antidepressants, azaspirodecanediones, barbituates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, 5-hydroxytryptamine antagonists, drugs for the treatment of hyperlipoproteinemia, hypnotics and sedatives, immunosupressive agents, laxatives, methylxanthines, monoamine oxidase inhibitors, neuromuscular blocking agents, organic nitrates, opioid analgesics and antagonists, pancreatic enzymes, phenothiazines, progestins, prostaglandins, agents for the treatment of psychiatric disorders, retinoids, sodium channel blockers, agents for spasticity and acute muscle spasms, succinimides, thioxanthines, thrombolytic agents, thyroid agents, tricyclic antidepressants, inhibitors of tubular transport of organic compounds, drugs affecting uterine motility, vasodilators, vitamins and the like, alone or in combination.

For use as a drug reservoir in a transdermal drug delivery device the polyurethane preferably satisfies the following criteria:
1) Sufficient flexibility such that the matrix material exhibits a modulus within the range of about 0.1MPa - 100 MPa at room temperature, preferably 0.5 -10 Mpa;
2) Sufficient drug and/or permeation enhancer loading at a matrix thickness of about 1-12 mils, preferably 2 - 6 mils such that a therapeutically effective amount of drug is delivered from the device to an individual throughout the administration period without delamination of any of the device layers; and
3) Process temperature of less than about 150 °C, preferably less than about 100 °C, and most preferably about 40 - 90 °C without the use of organic solvents.

The polyurethanes suitable for practice of this invention are the reaction product of aliphatic organic diisocyanates, high molecular weight polyether polyols, and low molecular weight glycols and may be prepared according to methods known in the art as disclosed in, for example, U.S. Patent No. 4,523,005. Polyurethanes suitable for practice of this invention include those provided by Thermedics, Inc. of Woburn, MA under the tradename Tecoflex® SG 80A, EG 80A, EG 85A, 24B, 38C, 578, 163C, 166A, LM 50D, and LM 70A. Tecoflex LM 70A is the preferred polyurethane for use in this invention.

Solubilities of the permeation enhance GML and laurydone in representative Tecoflex® polyurethanes are given below. Solubility in EVA is included for comparison

**Table 1**

| Tecoflex® Polyurethane | Solubility of GML | Solubility of laurydone |
|---|---|---|
| 24B | 5-7 wt% | <15 wt% |
| 38C | 6-8 wt% | <13 wt% |
| 163C | <4 wt% | 20-22 wt% |
| 166A | 4-5 wt% | 15-20 wt% |
| 57B | 6-8 wt% | 13-16 wt% |
| LM-70A | 5-6 wt% | > 12 wt% |

| Control | | |
|---|---|---|
| EVA | 2-3wt% | 10-14 wt% |

By manipulating the ratio of the hard and soft components of the polyurethane, a range of permeabilities for a given drug can be obtained for the polyurethane materials. Thus, the polyurethane material can be tailored to control the rate of release of drug from the device. For example, the ratio of the diisocyanate to the polyol can be varied by changing the amount of polyol in order to yield materials with different processibilities and diffusivities. In this manner, drug delivery devices which provide different delivery rates for the same or different drugs can be produced. If the ratio is high, the diffusivity is low and vice versa. The process temperature can be lowered by incorporating a second chain extender into the polyurethane formulation that disrupts the crystallinity formed by the hard segment (i.e. HDMI). Such chain extenders should also be compatible with the drug and any excipients such as permeation enhancers, for example, so that sufficient loadings of drug and / or excipients in the drug reservoir layer may be achieved.

By changing the nature of the soft segment, polyurethanes having different hydrophobic / hydrophilic character or drug solubility can be obtained in order to accommodate different drugs.

A preferred soft segment monomer is propylene glycol. Polyurethanes of intermediate hydrophilicity are obtained by either blending or copolymerizing two types of soft segment monomers. The polyurethane used in the method of the present invention has a process temperature of less than about 150 °C, preferably less than about 100 °C, most preferably between about 40 - 90 °C and a modulus value of between about 0.1 - 100 MPa (at room temperature). Preferred polyurethanes comprise polyether alcohols as the soft segment and a diisocyanate as the hard segment, and a diol as a chain extender. Preferred polyether alcohols include PTMEG having a molecular weight within the range of about 1000 - 2000 Daltons, and PPG. PPG is the particularly preferred soft segment as it provides polyurethanes that are more stable under elevated temperatures of greater than approximately 150 °C. 1,4 butane diol is a preferred chain extender, and HMDI is the preferred hard segment.

When used as the drug reservoir for a transdermal drug delivery device, the polyurethane polymer comprises about 50 - 98 wt%, preferably 75 - 95 wt% of the total weight of the drug reservoir.

The polyurethane drug reservoir contains 0.1 -40 wt%, preferably 1 - 20 wt %, of a drug to be delivered and 0 - 40 wt%, preferably 2 - 20 wt% of a permeation enhancer.

The transdermal drug delivery devices are preferably for administering fentanyl. Devices for the transdermal administration of fentanyl are known in the art as disclosed in, for example, U.S. Patent Nos. 4,470,962; 4,588,580; 4,626,539; 4,906,463; 4,911,916; 5,006,342; 5,186,939; 5,474,783; 5,762,952; and JP 142210, which are hereby incorporated in their entirety by reference.

A transdermal drug delivery device for administering fentanyl comprises:
a) a backing layer;
b) a drug reservoir comprising about 75 - 95 wt% of a polyether polyurethane having a process temperature of less than 150 °C, preferably less than 100 °C, most preferably about 40 - 90 °C, wherein the polyurethane drug reservoir layer contains:
   i) 1 - 10 wt%, preferably 3 - 8 wt%, most preferably 5 - 7 wt% fentanyl base;
   ii) 0 - 20 wt% of a permeation enhancer, preferably 2 - 17 wt% permeation enhancer, and most preferably 4 - 15 wt% of a permeation enhancer, and
c) means for maintaining the device in fentanyl transmitting relationship with a body surface or membrane.

The preferred polyurethane is Tecoflex® LM 70A available from Thermedics of Woburn MA.

I t is preferable to provide the drug reservoir with fentanyl at or below saturation, most preferably below saturation in order to avoid phase separation. It is also preferable to provide permeation enhancer at or below saturation in the drug reservoir.

The devices are preferably multilaminate devices comprising an in-line pressure sensitive adhesive as the means for maintaining the device in fentanyl transmitting relationship with a body surface or membrane. Preferably, the solubility of the adhesive for fentanyl and any permeation enhancer is less than the solubility of the drug reservoir for fentanyl and any permeation enhancer. Preferably, the adhesive contains less than about 5 wt% fentanyl after equilibration. Acrylate pressure sensitive adhesives are preferred. Examples of acrylate adhesives include NS87-2100, NS87-2287 and NS87-2516 provided by National Starch and Chemical Co. NS87-2287 is a solution polyacrylate comprising vinyl acetate, 2-ethylhexyl acrylate, hydroxyethyl acrylate, and glyadyl methacrylate monomers and is the preferred adhesive according to this embodiment. It contains no crosslinking agent and is available as a 50% solids solution in ethyl acetate.

The backing layer is preferably medium density polyethylene (MDPE) / polyester / aluminum / ethylene vinylacetate (EVA) laminates or a low density polyethylene / polyurethane laminate. Polyolefin / polyurethane films adhere well to the polyurethane drug reservoirs described herein, while medium density polyurethane (MDPE)/polyester/aluminum / ethylene vinylacetate (EVA) laminates have been observed to yield slightly higher flux with slightly less adhesion to the polyurethane reservoir.

Preferred permeation enhancers for administering fentanyl include monoglycerides and lauryl pyroglutamate. Lauryl pyroglutamate may be obtained from DD Chemco (Northridge, CA) under the tradename Laurydone® (U.C.I.B., Anet, France).

Having thus generally described our invention, the following specific examples describe preferred embodiments thereof but are not intended to limit the invention in any manner.

### Example 1

Drug reservoirs were prepared by mixing fentanyl base, permeation enhancer, and polyurethane granules in a Brabender mixer (30 cc) provided with a heater in order to melt-mix the formulations. After mixing for approximately 30 minutes, the drug reservoir formulation was calendered into a 5 mil thick film. The film was then laminated to a backing layer (Medpar®, 3M, St. Paul, MN) which was subsequently laminated to an acrylate adhesive layer (NS87-2287, National Starch and Chemical Co., Bridgewater, NJ). Circular systems having a 2.54 cm² surface area were punched. The mix had a process temperature of approximately 65 °C. System compositions are shown in Table 2. A Duragesic® (Janssen Pharmaceuticals) system prepared according to the method set forth in Example 1 of U.S. Patent No. 4,588,580 was used as the control and had its surface area outside 2.54 cm² masked to normalize surface area available for drug delivery.

**Table 2**

| Drug/Permeation Enhancer Reservoir Composition | | |
|---|---|---|
| Sample | Formulation | Weight % |
| 1 | Fentanyl/ laurydone/ polyurethane | 6/15/79 |
| 2 | Fentanyl/ laurydone/ polyurethane | 6/10/84 |
| 3 | Fentanyl/laurydone/GML/polyurethane | 6/7.5/2.5/84 |
| 9 | control | |

Circular pieces of human epidermis were placed with stratum corneum facing up. The release liner of the laminate was removed and the fentanyl releasing side of the system was centered over the stratum corneum side of the epidermis which had been blotted dry just prior to use. The edges of epidermis were then folded around the system so that none of the system edge was exposed to the receptor solution. This assembly was then mounted on a Teflon® holder of a release rate rod using nylon mesh and metal string. A known volume of receptor solution (0.05M KH₂PO₄ / K₂HPO₄, pH 6.5) was then placed in a test tube and was equilibrated at 35°C. The test tube was placed in a water bath and maintained at 35°C. The Teflon rod with system and epidermis attached was then reciprocated within the test tube by attaching the rod to a motor which caused constant vertical mixing.

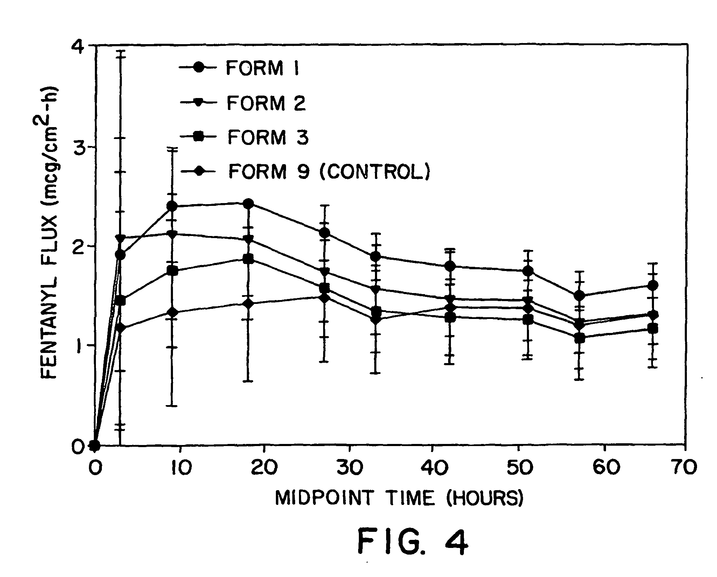

At given time intervals, the entire receptor solution was removed from the test tubes and replaced with an equal volume of fresh receptor solutions previously equilibrated at 35°C. The receptor solutions are stored in capped vials at 4 °C until assayed for fentanyl content by HPLC. From the drug concentration and the volume of the receptor solutions, the area of permeation and the time interval, the flux of the drug through the epidermis was calculated as follows: (drug concentration X volume of receptor)/(area x time) = flux (µg/cm² · hr). The *in vitro* flux of fentanyl through epidermis at 35 °C is shown in Figure 4.

## Claims

1. A method of making a reservoir matrix material for a transdermal drug delivery device, which method comprises the steps of:
(a) providing a drug;
(b) providing a polyurethane polymer; and
(c) melt-blending the drug into the polyurethane polymer at a temperature of less than 150°C.

2. A method according to claim 1 wherein the drug and polyurethane polymer are melt-blended at a temperature of less than 100°C.

3. A method according to claim 2 wherein the drug and polyurethane polymer are melt-blended at a temperature of 40 - 90°C.

4. A method according to claim 1, 2 or 3 wherein the polyurethane polymer is a polyether polyurethane.

5. A method according to claim 4 wherein the polyurethane polymer comprises the reaction product of at least one aliphatic diisocyanate; at least one high molecular weight polyether polyol, and at least one low molecular weight glycol.

6. A method according to claim 5 wherein the diisocyanate comprises methylene bis (cyclohexyl) diisocyanate, and the polyether polyol comprises polytetramethylene ether glycol, polypropylene glygol, or polyethylene glycol.

7. A method according to claim 5 or 6 wherein the glycol is 1,4-butane diol.

8. A method according to claim 6 or 7 wherein the polyether polyol is a mixture of at least two polymers selected from the group consisting of polytetramethylene ether glycol, polypropylene glycol, polyethylene glycol and propylene glycol.

9. A method according to any preceding claim wherein the matrix material has a thickness of 25.4 to 304.8 µm (1 to 12 mils).

10. A method according to claim 9 wherein the matrix material has a thickness of 50.8 to 152.4 µm (2 to 6 mils).

11. A method according to any preceding claim wherein the matrix material has a room-temperature modulus between 0.1 and 100 MPa.

12. A method according to any preceding claim wherein the matrix material further contains at least one permeation enhancer in an amount of up to 20 wt%.

13. A method according to claim 12 wherein the matrix material further contains 2 - 15 wt% of a permeation enhancer.

14. A method according to claim 12 or 13 wherein the permeation enhancer is selected from the group consisting of monoglycerides and lauryl pyroglutamate.

15. A method according to claim 14 wherein the permeation enhancer is glycerol monolaurate, or lauryl pyroglutamate.

16. A method according to any preceding claim wherein the matrix material comprises 0.1 - 40 wt% of the drug.

17. A method according to any preceding claim wherein the drug is selected from the group consisting of fentanyl, oxbutynin, and fluoxetine.

18. A method according to claim 17 wherein the matrix material contains 1 - 10 wt% fentanyl base.

19. A method according to claim 18 wherein the matrix material contains 4 - 7 wt% fentanyl base, 4 - 13 wt% of a permeation enhancer, and 75 - 92 wt% of a polyether polyurethane.

## Patentansprüche

1. Verfahren zur Herstellung eines Reservoirmatrixmaterials für eine transdermale Medikamentenabgabevorrichtung, wobei das Verfahren die folgenden Schritte umfasst:
(a) Bereitstellen eines Medikaments;
(b) Bereitstellen eines Polyurethanpolymers; und
(c) Schmelzmischen des Medikaments in das Polyurethanpolymer bei einer Temperatur unterhalb von 150°C.

2. Verfahren nach Anspruch 1, worin das Medikament und das Polyurethanpolymer bei einer Temperatur unterhalb von 100°C schmelzgemischt werden.

3. Verfahren nach Anspruch 2, worin das Medikament und das Polyurethanpolymer bei einer Temperatur von 40-90°C schmelzgemischt werden.

4. Verfahren nach Anspruch 1, 2 oder 3, worin das Polyurethan polymer ein Polyetherpolyurethan ist.

5. Verfahren nach Anspruch 4, worin das Polyurethanpolymer das Reaktionsprodukt aus mindestens einem aliphatischen Diisocyanat, mindestens einem Polyetherpolyol mit hoher Molmasse und mindestens einem Glycol mit niedriger Molmasse umfasst.

6. Verfahren nach Anspruch 5, worin das Diisocyanat Methylenbis(cyclohexyl)diisocyanat, und das Polyetherpolyol Polytetramethylenetherglycol, Polypropylenglycol oder Polyethylenglycol umfasst.

7. Verfahren nach Anspruch 5 oder 6, worin das Glycol 1,4-Butandiol ist.

8. Verfahren nach Anspruch 6 oder 7, worin das Polyetherpolyol ein Gemisch aus mindestens zwei Polymeren ist, ausgewählt aus der Gruppe, die aus Polytetramethylenetherglycol, Polypropylenglycol, Polyethylenglycol und Propylenglycol besteht.

9. Verfahren nach einem der vorhergehenden Ansprüche, worin das Matrixmaterial eine Dicke von 25,4 bis 304,8 µm (1 bis 12 mils) hat.

10. Verfahren nach Anspruch 9, worin das Matrixmaterial eine Dicke von 50,8 bis 152,4 µm (2 bis 6 mils) hat.

11. Verfahren nach einem der vorhergehenden Ansprüche, worin das Matrixmaterial ein Raumtemperaturmodul zwischen 0,1 und 100 MPa hat.

12. Verfahren nach einem der vorhergehenden Ansprüche, worin das Matrixmaterial weiterhin mindestens einen Permeationsverstärker in einer Menge von bis zu 20 Gew.-% enthält.

13. Verfahren nach Anspruch 12, worin das Matrixmaterial weiterhin 2-15 Gew.-% eines Permeationsverstärkers enthält.

14. Verfahren nach Anspruch 12 oder 13, worin der Permeationsverstärker ausgewählt ist aus der Gruppe, die aus Monoglyceriden und Laurylpyroglutamat besteht.

15. Verfahren nach Anspruch 14, worin der Permeationsverstärker Glycerinmonolaurat oder Laurylpyroglutamat ist.

16. Verfahren nach einem der vorhergehenden Ansprüche, worin das Matrixmaterial 0,1-40 Gew.-% des Medikaments umfasst.

17. Verfahren nach einem der vorhergehenden Ansprüche, worin das Medikament ausgewählt ist aus der Gruppe, die aus Fentanyl, Oxbutynin und Fluoxetin besteht.

18. Verfahren nach Anspruch 17, worin das Matrixmaterial 1-10 Gew.-% Fentanylbase enthält.

19. Verfahren nach Anspruch 18, worin das Matrixmaterial 4-7 Gew.-% Fentanylbase, 4-13 Gew.-% eines Permeationsverstärkers und 75-92 Gew.-% eines Polyetherpolyurethans enthält.

## Revendications

1. Procédé de fabrication d'une matière de matrice de réservoir pour un dispositif d'administration transdermique de médicament, lequel procédé comprend les étapes consistant à :
(a) prendre un médicament ;
(b) prendre un polyuréthane polymère ; et
(c) incorporer par mélange à l'état fondu le médicament dans le polyuréthane polymère à une température de moins de 150°C.

2. Procédé selon la revendication 1, dans lequel le médicament et le polyuréthane polymère sont mélangés à l'état fondu à une température de moins de 100°C.

3. Procédé selon la revendication 2, dans lequel le médicament et le polyuréthane polymère sont mélangés à l'état fondu à une température de 40 - 90°C.

4. Procédé selon l'une des revendications 1, 2 ou 3, dans lequel le polyuréthane polymère est un polyéther polyuréthane.

5. Procédé selon la revendication 4, dans lequel le polyuréthane polymère comprend le produit de réaction d'au moins un diisocyanate aliphatique, d'au moins un polyéther polyol de masse moléculaire élevée, et d'au moins un glycol de faible masse moléculaire.

6. Procédé selon la revendication 5, dans lequel le diisocyanate comprend le méthylène bis (cyclohexyl) diisocyanate, et le polyéther polyol comprend le polytétraméthylène éther glycol, le polypropylène glycol ou le polyéthylène glycol.

7. Procédé selon l'une des revendications 5 où 6, dans lequel le glycol est le 1,4-butane diol.

8. Procédé selon l'une des revendications 6 ou 7, dans lequel le polyéther polyol est un mélange d'au moins deux polymères choisis dans le groupe constitué par le polytétraméthylène éther glycol, le polypropylène glycol, le polyéthylène glycol et le propylène glycol.

9. Procédé selon l'une quelconque des revendications précédentes, dans lequel la matière de matrice a une épaisseur de 25,4 à 304,8 µm (1 à 12 mils).

10. Procédé selon la revendication 9, dans lequel la matière de matrice a une épaisseur de 50,8 à 152,4 µm (2 à 6 mils).

11. Procédé selon l'une quelconque des revendications précédentes, dans lequel la matière de matrice a un module à température ambiante entre 0,1 et 100 MPa.

12. Procédé selon l'une quelconque des revendications précédentes, dans lequel la matière de matrice contient en outre au moins un agent augmentant la pénétration dans une quantité allant jusqu'à 20% en poids.

13. Procédé selon la revendication 12, dans lequel la matière de matrice contient en outre 2 - 15% en poids d'un agent augmentant la pénétration.

14. Procédé selon l'une des revendications 12 ou 13, dans lequel l'agent augmentant la pénétration est choisi dans le groupe constitué par les monoglycérides et le pyroglutamate de lauryle.

15. Procédé selon la revendication 14, dans lequel l'agent augmentant la pénétration est le monolaurate de glycérol ou le pyroglutamate de lauryle.

16. Procédé selon l'une quelconque des revendications précédentes, dans lequel la matière de matrice comprend 0,1 - 40% du poids du médicament.

17. Procédé selon l'une quelconque des revendications précédentes, dans lequel le médicament est choisi dans le groupe constitué par le fentanyle, l'oxbutynine et la fluoxétine.

18. Procédé selon la revendication 17, dans lequel la matière de matrice contient 1 - 10% en poids de fentanyle base.

19. Procédé selon la revendication 18, dans lequel la matière de matrice contient 4 - 7% en poids de fentanyle base, 4 - 13% en poids d'un agent augmentant la pénétration, et 75 - 92% en poids d'un polyéther polyuréthane.
